# EUROPEAN PATENT APPLICATION

(11) **EP 1 955 689 A1**
(43) Date of publication of application: **13.08.2008**
(21) Application number: 08250493.7
(22) Date of filing: 11.02.2008
(51) Int. Cl.: A61K 8/00, A61K 9/70

(54) **Positioning feature for aiding use of film or strip product**

(30) Priority: 12.02.2007 US 705263
(71) Applicant: McNeil-PPC, Inc., Skillman, NJ 08558 (US)
(72) Inventor: Georgiades, Constantine, East Brunswick, NJ 08816 (US); Borodin, Alexander, Monmouth Junction, NJ 08852 (US); Costello, Peter, Franklin Lakes, NJ 07417 (US); Lepage, Jacqueline, Boonton, NJ 07005 (US); Schaefer, Wolfgang, Madison, NJ 07940 (US); Krumme, Markus, Denville, NJ 07834 (US)
(74) Representative: Kirsch, Susan Edith

(57) **Abstract**

A film or strip product is provided with a positioning feature formed to aid a user in differentiating or distinguishing or otherwise identifying one or more features of the film or strip product. The positioning feature thus may be used to facilitate proper or desired use of the film product upon identification of a desired feature thereof. In one embodiment, the positioning feature is configured to aid in properly orienting and/or positioning and/or applying the film or strip product. The film or strip product may provide a therapeutic affect to a desired treatment site, and may require positional orientation before application to the treatment site, and/or positional orientation with respect to the treatment site. Various positioning features that may serve such function include visual or tactile features that either visually or tactilely indicate to a user sufficient information about the film product to permit the user to use and/or apply and/or orient the film product as desired or required.

## Description

### FIELD OF THE INVENTION

The present invention relates to a positioning feature on a film or strip type product, the positioning feature being provided to differentiate or distinguish features of the film or strip type product. More particularly, the present invention relates to a positioning feature that assists a user in orienting and/or positioning and/or applying a film or strip type product configured to provide a therapeutic effect at a selected body area. Even more particularly, the film or strip type product of the present invention may carry an active for delivery to the desired body area for treatment of such body area.

### BACKGROUND OF THE INVENTION

A variety of topically-applied products, including strips, films, patches and the like, are known in the art. Such products are particularly useful where a protectant film is recommended or where drug or medication retention is desirable.

Film protectants are particularly desirable in situations where wounds or surface openings are present and must be protected. Alternatively, where a drug or medication is easily removed by rinsing or wiping the application area (e.g., transdermal applications), mechanical retention of the drug or medication becomes particularly desirable.

Most recently, strip or film type products have enjoyed renewed popularity in the oral care field. Particular interest has been paid to the areas of teeth whitening and oral transdermal delivery of drugs and medications.

Although a variety of strip or film type products have been disclosed, there still remains a need for improved film or film-like compositions which reduce the inconvenience or discomfort typically associated with the attachment of such foreign objects to sensitive parts of the body. There also remains a need for improved film or film-like compositions which are easier to use and to apply.

For instance, one disadvantage observed regarding the aforementioned film or strip products relates to the eventual need to peel off or in some other way to remove and to discard the film or strip product after delivery of the topical or systemic active.

A related disadvantage is that typically an adhesive material is required to maintain the film or strip product on the desired area until treatment is complete. Such adhesive material may leave a residue at the treatment site. Moreover, when an adhesive material is provided, generally a releasable backing strip is also provided to protect the adhesive material from accumulating debris and also to prevent the film or strip product from adhering to the wrong object or to itself. Such releasable backing strip must be peeled off so that the film or strip product may be adhered to the desired body area for delivery of the active, thereby adding additional inconveniences, such as the need to remove and discard an element, and the need to prevent adherence of the film or strip product to itself.

The inventors of United States patent applications 11/086,517, 11/030,846, and 10/792,362 have discovered that film compositions comprising select water insoluble polymers and a disintegration facilitator selected from the group consisting of a plasticizer, a water insoluble particulate, or mixtures thereof is one way to address issues around "peeling" since the above ingredients provide film compositions having good protective properties as well as improved disintegration properties. Such film compositions may be formed into a film product that facilitates use of the product and application thereof to a desired body area to deliver an active, such as a systemic or topical active, to the desired body area.

The film products disclosed in United States patent applications 11/086,517, 11/030,846, and 10/792,362 may have protective properties such that the film prevents foreign substances, chemicals, or actives from crossing from one side of the film to the other. Additionally, or alternatively, the disclosed film products may have controlled (or an extended type or prolonged) disintegration or dissolution properties in aqueous environments. For instance, the film product may deliver the topical or systemic active and thereafter dissolve within a predetermined amount of time, such as within 60 minutes, optionally within 45 minutes, optionally, within 30 minutes or, optionally, within 15 minutes in an aqueous environment.

However, it is possible to form such film products such that a dry adhesive layer is provided such that no releasable backing strip is required. As such, the surface bearing the active may not be readily distinguishable from the opposite surface which may not comprise and/or deliver an active. In such case, it would be desirable to differentiate the active-bearing surface from the inactive surface without affecting the ease with which the film product may be applied or used. It will be appreciated that other types of film products with functionally different surfaces that cannot readily be distinguished from each other are available with similar inconveniences associated with use and application of the correct surface.

### SUMMARY OF THE INVENTION

In accordance with the principles of the present invention, a positioning feature is provided on a film or strip product (hereinafter, simply "film product(s)" for the sake of simplicity and without intent to limit such term) to aid in differentiating or distinguishing features of the film product. Differentiation of features of a film product is helpful for various purposes, including, without limitation, facilitating orientation of the film product. A particular orientation of the film product may be necessary such as for proper application and/or delivery of an active carried by the film product to a desired body area.

According to one aspect of the invention, the film product may be formed such that only one surface thereof is configured to deliver or to apply an active or such that one side delivers one active and the opposite side delivers a second active optionally different in identity and function from the first active. As such, the positioning feature is provided to indicate to a user the proper or appropriate surface to apply to the treatment site so that the active may be delivered to such site.

The positioning feature may be in any desired form capable of achieving its purpose of distinguishing features of the film product, such as for purposes of orienting the film product. For instance, an irregularity in shape or texture or visual feature may be provided along the film product. If the film product is symmetrical about a midline, then the positioning feature may be provided at a position offset from the midline to distinguish the surfaces of the film product from each other.

These and other features and advantages of the present invention will be readily apparent from the following detailed description of the invention, the scope of the invention being set out in the appended claims.

### BRIEF DESCRIPTION OF THE DRAWINGS

The detailed description will be better understood in conjunction with the accompanying drawings, wherein like reference characters represent like elements, as follows:

FIGURE 1 is a plan view of an exemplary film product having an exemplary positioning feature in accordance with the principles of the present invention;

FIGURE 2 is a plan view of a film product similar to that of FIGURE 1, but with a different exemplary positioning feature;

FIGURE 3 is a plan view of a film product similar to that of FIGURE 1, but with a different exemplary positioning feature;

FIGURE 4 is a plan view of a film product similar to that of FIGURE 1, but with a different exemplary positioning feature;

FIGURE 5 is a plan view of another exemplary film product having an exemplary positioning feature similar to that of FIGURE 3; and

FIGURE 6 is a perspective view of yet another exemplary film product, with a another type of exemplary positioning feature.

### DETAILED DESCRIPTION OF THE INVENTION

In accordance with the principles of the present invention, an indicator or positioning feature (hereinafter "positioning feature" for the sake of convenience, without intent to limit such term) is provided on a product that is to be applied to a desired treatment site or body area (hereinafter "treatment site" for the sake of convenience, without intent to limit such term). Positioning features formed in accordance with the principles of the present invention are particularly configured to aid the user in differentiating or distinguishing features of the film product. A further purpose of such positioning features may be to aid the user in orienting the product so that the product may perform its desired treatment function effectively. Such positioning features are particularly important, if not necessary, on products that cannot perform their desired functions unless properly positioned or oriented in a particular manner. However, such positioning features are helpful even if not essential to the proper functioning of the product. Products utilizing the positioning features of the present invention are referenced herein as "film products" for the sake of convenience, such term being used in the broad sense to include not only films, but also strips, patches, pads, layers, wafers, and the like, and should not be understood as necessarily limited to film-like properties.

A common function of film products modified by the present invention is to perform a therapeutic function at a selected treatment site in any of a variety of manners. Therapeutic functions include, without limitation, providing a barrier function (such as covering the site to protecting it from contamination or injury, such as by the use of a bandage); or delivering an active, either systemically (such as transcutaneous deliver of a medicament) or topically (such as application of a bleaching agent to teeth, or an antibiotic or analgesic to a dermal wound). The film product may itself have to be positioned in a particular orientation before application to the treatment site, or may have to be applied in a particular orientation with respect to the treatment site.

The film products of the present invention can comprise, consist of, or consist essentially of the essential elements and limitations of the film products disclosed in United States patent applications 11/086,517, 11/030,846, and 10/792,362, as well as any desirable additional or alternative or optional ingredients, components, or limitations known in the art.

Exemplary film products modified by the present invention are disclosed in United States patent applications 11/086,517, 11/030,846, and 10/792,362 and are incorporated herein by reference and described in detail hereafter.

### The Water Insoluble Polymer

The film compositions comprise water insoluble polymers. Suitable water insoluble polymers include, but are not limited to, hydrogenated vegetable oils; natural rosins such as wood rosins and gum rosins; vegetable proteins such as corn protein, pea protein or soy protein; hydrogenated caster oil; polyvinyl chloride; shellac; polyurethane; cellulose, cellulose derivatives such as cellulose or ethylcellulose; waxes; polymers such as those sold under the Trade Mark Eudragit RS (ammoniomethacrylate copolymer), silicone or a mixture thereof.

Hydrogenated vegetable oils suitable for use herein include, but are not limited to, hydrogenated forms of safflower oil, caster oil, coconut oil, cottonseed oil, canola oil, menhaden oil, palm kernel oil, palm oil, peanut oil, soybean oil, rapeseed oil, linseed oil, rice bran oil, pine oil, sesame oil, sunflower seed oil, hydrogenated safflower oil and mixtures thereof.

Examples of the waxes suitable for use herein include, but are not limited to, paraffin, carnauba wax, candelilla wax, sugarcane wax, beeswax, cetyl esters wax, montan wax, glycowax, castor wax, spermaceti wax, shellac wax, microcrystalline wax, vaseline and mixtures thereof.

Eudragit polymers are polymeric lacquer substances based on acrylate and methacrylate. Polymers sold under the Trademark Eudragit RL and RS are resins comprising copolymers of acrylic and methacrylic acid esters with a low content of quaternary ammonium groups and are described in the "Eudragit" brochure of Rohm Pharma GmbH (1982) wherein detailed physical- chemical data of these products is given. The ammonium groups are present as salts and give rise to permeability of the lacquer films. Eudragit RL and RS are freely permeable (RL) or slightly permeable (RS), respectively independent of pH. Additional water insoluble polymers are detailed in US patents 6,183,777; 4,721,619; and 6,251,427.

Mixtures of any of the above ingredients can also be used.

In certain embodiments, the water insoluble polymer can include shellac sold under the name Pharmaceutical Glaze and supplied by Mantrose Haeser Co., Attleboro, Ma.

When incorporated in the film compositions, the water insoluble polymer is present at a concentration of from about 10% to about 80%, optionally, from about 15% to about 40%, and, optionally, from about 20% to about 35%, by weight, of the wet film composition.

### The Disintegration Facilitator

### Plasticizers or Plasticizing Agents

The film compositions may optionally also comprise at least one disintegration facilitator selected from the group consisting of plasticizers or plasticizing agents, water insoluble particles or mixtures thereof.

Examples of suitable plasticizers include, but are not limited to, citric acid alkyl esters, glycerol esters such as glycerol monooleate and glycerol monostearate, phthalic acid alkyl esters, sebacic acid alkyl esters, sucrose esters, sorbitan esters, acetylated monoglycerides, glycerols, fatty acid esters, glycols, propylene glycol, and polyethylene glycols 200 to 12,000 and mixtures thereof. Specific plasticizers include, but are not limited to, lauric acid, sucrose, sorbitol, triethyl citrate, acetyl triethyl citrate, triacetin (glyceryl triacetate), poloxamers, alkyl aryl phosphates, diethyl phthalate, tributyl citrate, dibutyl phthalate, dibutyl sebacate, polysorbate, Carbwax® series of polyethylene glycols (Union Carbide Corporation) and mixtures thereof.

In certain embodiments, the plasticizers can include mono- and di-glycerides of edible fats or oils supplied by Lonza Inc., Fair Lawn, NJ or Eastman Triacetin (food grade) supplied by Eastman Chemical Company, Kingsport, TN.

When incorporated in the film compositions, the plasticizer is present at a concentration of from about 0.1 % to about 10%, preferably from about 0.1 % to about 5%, and most preferably from about0.5% to about 1.5% by weight of the wet film composition.

### Water Insoluble Particles

The optional disintegration facilitator can also be a water insoluble particle. Various kinds of organic powders and inorganic powders can be used as the water-insoluble particles.

The inorganic powders which are useful herein include, but are not limited to, microfine particles or granules of alumina, talc, magnesium stearate, titanium dioxide, barium titanate, magnesium titanate, calcium titanate, strontium titanate, zinc oxide, silica sand, clay, mica, tabular spar, diatomaceous earth, various inorganic oxide pigments, chromium oxide, cerium oxide, iron red, antimony trioxide, magnesium oxide, zirconium oxide, barium sulfate, barium carbonate, calcium carbonate, silica (colloidal or fumed), silicon carbide, silicon nitride, boron carbide, tungsten carbide, titanium carbide, carbon black and mixtures thereof.

The organic powders which are useful herein include cross- linked and non-cross-linked polymer powders, organic pigments, charge controlling agents, and waxes, for example. The cross-linked and non- cross-linked resin powders include, but are not limited to, resin powders of the styrene type, acrylic type, methacrylic type, polyethylene type, polypropylene type, silicone type, polyester type, polyurethane type, polyamide type, epoxy type, polyvinyl butyral type, rosin type, terpene type, phenol type, melamine type, and guanamine type, for example. Mixtures of any of the above organic or inorganic powders can also be used. Additional useful particles can be found in US patents 6,475,500; US 5,611,885; and US 4,847,199.

The water insoluble particles generally have a particle size of less than 10 microns, optionally, from about 0.01 microns to about 5 microns, optionally, from about 0.1 microns to about 1 micron, and, optionally, from about 0.1 to about 0.5 microns.

In certain embodiments, the insoluble particles can include Cabosil M-5 (fumed untreated silica) supplied by Cabot, Tuscola, III.

When incorporated in the film compositions, the water insoluble particle is present at a concentration of from about 0.1% to about 20%, optionally, from about 0.5% to about 15%, and, optionally, from about 1% to about 10% by weight of the wet film composition.

### pH-Dependent Water Hydratable Polymer

The compositions may also optionally comprise pH-dependent water hydratable polymers. The pH-dependent water hydratable polymers are copolymers derived from (i) at least one anionic monomer comprising at least one carboxylic group, and (ii) at least one neutral non-dissociating monomer. The term "pH-dependent water hydratable polymers", as used herein means water hydratable polymers the solubility of which increases as pH increases.

Suitable anionic monomers include, but are not limited to, acrylic acid, methacrylic acid, maleic acid, fumaric acid, and itaconic acid. Suitable neutral monomers include, but are not limited to, C₁-C₂₀ alkyl acrylate, C₁-C₂₀ alkyl methacrylate, C₁-C₂₀ alkyl carboxylic acid vinyl ester, C₁-C₂₀ alkyl vinyl ether, C₁-C₂₀ alkene, styrene, ester derived from a carboxy C₂-C₅ alkene and a polyethyleneglycol(1-30) monosubstituted with C₁-C₃₀ alkyl group, the structure of which is illustrated by the formula 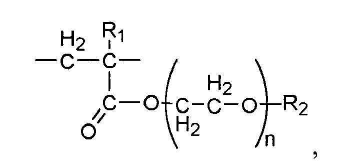 where R₁ is hydrogen or C₁-C₃ alkyl, R₂ is C₁-C₃₀ alkyl, and n is from 1 to 30.

In certain embodiments, the molar ratio of anionic monomers to neutral monomers is from about 1:10 to about 10:1, optionally from about 1:5 to about 5:1, optionally from about 1:2 to about 2:1.

The pH-dependent water hydratable polymers suitable for use in the present invention include polymers which are essentially insoluble below about pH 5.5, such as ethyl acrylate/methacrylic acid copolymers, methyl methacrylate/methacrylic acid copolymers, methacrylic acid/methyl acrylate/methyl methacrylate copolymers and the like, and mixtures thereof. pH-dependent polymers incorporated in certain embodiments include, but are not limited to, ethyl acrylate/methacrylic acid copolymers wherein the ratio of free carboxyl groups to esters is about 1:1 (Eudragit® L 30 D, solubilization pH>5.5, available from Röhm Pharma GmbH, Weiterstadt, Germany; and Kollicoat® MAE 30 D, solubilization pH>5.5, available from BASF, Ludwigshafen, Germany), methyl methacrylate/methacrylic acid copolymers wherein the ratio of free carboxyl groups to esters is from about 1:1 to about 1:2 (Eudragit® S100, 1:2 ratio, solubilization pH>7.0, available from Röhm Pharma; and Eudragit® L100, 1:1 ratio, solubilization pH>6.0, available from Röhm Pharma), methacrylic acid/methyl acrylate/methyl methacrylate copolymers wherein the ratio of methacrylic acid, methyl acrylate and methyl methacrylate monomers is about 1:5:2 to 3:7:3 (Preparation 4110D, 1:6.5:2.5 ratio, solubilization pH>7.2, available from Röhm Pharma), and mixtures thereof. Also useful here is Eudragit L30 D55, available from Röhm Pharma.

Also useful as a pH-dependent water hydratable polymer are copolymers derived from (i) at least one monomer comprising at least one ester derived from a carboxylic acid and a polyethylene glycol ether wherein the polyethylene glycol ether comprises at least one fatty chain comprising more than 18 carbon atoms, and (ii) at least one monomer comprising at least one carboxylic acid group. The at least one monomer comprising at least one carboxylic acid group, in one embodiment, may be chosen from acrylic acid and methacrylic acid. The at least one additional anionic associative polymer may further comprise at least one unit comprising at least one ester chosen from esters derived from acrylic acid and a polyethylene glycol ether comprising at least one fatty chain comprising more than 18 carbon atoms, and esters derived from methacrylic acid and a polyethylene glycol ether comprising at least one fatty chain comprising more than 18 carbon atoms. The polyethylene glycol ether, for example, may be chosen from polyethylene glycol ethers of at least one alcohol chosen from nonadecanol, arachidyl alcohol, heneicosanol, behenyl alcohol, tricosanol, triacontanol, and hentriacontanol. Non-limiting examples of the at least one additional anionic associative polymer which may be used in the composition according to the present invention include Acrylates/Beheneth-25 Methacrylate Copolymer, which is sold by Rohm & Haas under the name Aculyn 28; copolymer of acid and acrylate comonomers, which is sold by Rohm & Haas under the name Aculyn 33; and terpolymer of acrylic acid, acrylate esters, and steareth-20 methacrylate ester, which is sold by Rohm & Haas under the name Aculyn 22.

When incorporated in the film compositions, the pH dependent water hydratable polymer is present at a concentration of from about 0.01 % to about 60%, optionally, from about 0.5% to about 20%, and, optionally, from about 1% to about 5% by weight of the wet film composition.

Where the film products (such as disclosed in United States patent applications 11/086,517, 11/030,846, and 10/792,362) form the first or backing layer of a multi-layer or bi-layer film, the thickness of this first or backing layer can optionally range from about 1 micron to about 20 microns, optionally from about 3 microns to about 15 microns, optionally from about 5 microns to about 12 microns. The thicknesses of any additional layers can equal the range of thickness of the first (or backing layer) or range from about 30 microns to about 150 microns, optionally from about 45 microns to about 130 microns, optionally from about 70 microns to about 120 microns.

### Optional Ingredients

Various topical and systemic actives can also be incorporated into the disclosed films. The term "topical or system active" as used herein includes curative, prophylactic and cosmetic active substances or compositions thereof. Examples of the conditions these substances may address include, but are not limited to one or more of, appearance and structural changes to teeth, whitening, stain bleaching, stain removal, plaque removal, tartar removal, cavity prevention and treatment, inflamed and/or bleeding gums, mucosal wounds, lesions, ulcers, aphthous ulcers, cold sores, tooth abscesses, tooth and/or gum pain, tooth sensitivity (e.g. to temperature changes), and the elimination of mouth malodour resulting from the conditions above and other causes such as microbial proliferation.. Additionally, the films of the present invention are useful for treating and/or preventing wounds, lesions, ulcers, cold sores and the like of the lips and skin generally.

Suitable topical actives for use in and around the oral cavity include any substance that is generally considered as safe for use in the oral cavity and that provides a change to the overall health of the oral cavity. The level of topical oral care active in the present invention may generally be from about 0.01 % to about 40% or, optionally, from about 0.1 % to 20% by weight of the wet film.

The topical oral care actives may include many of the actives previously disclosed in the art. The following is a non all- inclusive list of oral care actives that may be used.

Essential oils may be included in or associated with the films. Essential oils suitable for use herein are described in detail in US patents 6,596,298 to Leung et al..

Teeth whitening actives may be included in the films. The actives suitable for whitening are selected from the group consisting of oxalates, peroxides, metal chlorites, perforates, percarbonates, peroxyacids, and mixtures thereof. Suitable peroxide compounds include: hydrogen peroxide, calcium peroxide, sodium peroxide, carbamide peroxide, urea peroxide, sodium percarbonate and mixtures thereof. Optionally, the peroxide is hydrogen peroxide. Suitable metal chlorites include calcium chlorite, barium chlorite, magnesium chlorite, lithium chlorite, sodium chlorite and potassium chlorite. Additional whitening actives may be hypochlorite and chlorine dioxide. A preferred chlorite is sodium chlorite. The effectiveness of whitening actives can, optionally, be enhanced by means of a catalyst, i.e. a two-component peroxide- catalyst; system. Useful whitening agent catalysts or catalytic agents can be found in US 6, 440,396 to McLaughlin, Gerald.

When incorporating peroxide actives, the film compositions can, optionally, contain peroxide active stabilizers. Peroxide active stabilizers suitable for use herein include, but are not limited to polyethylene glycols such as PEG 40 or PEG 600; zinc salts such as zinc citrate; polyoxyalkylene block-polymers (e.g., Pluronics); aminocarboxylic acids or salts thereof; glycerols; dyes such as Blue #1 or Green #3; phosphates such as phosphoric acid, sodium phosphate or sodium acid pyrophosphate; stannous salts such as stannous chloride; sodium stannate; citric acid; etidronic acid; carbomers or carboxypolymethylenes such as those of the Carbopol® seriers, butylated hydroxytoluene (BHT), ethylenediaminetetraacetic acid (EDTA) and mixtures thereof.

Anti-tartar agents useful herein include: phosphates. Phosphates include pyrophosphates, polyphosphates, polyphosphonates and mixtures thereof. Pyrophosphates are among the best known for use in dental care products. Pyrophosphate ions delivered to the teeth derive from pyrophosphate salts. The pyrophosphate salts useful in the present compositions include the dialkali metal pyrophosphate salts, tetra-alkali metal pyrophosphate salts, and mixtures thereof. Disodium dihydrogen pyrophosphate (Na₂H₂P₂O₇), tetrasodium pyrophosphate (Na₄P₂O₇), and tetrapotassium pyrophosphate (K₄P₂O₇) in their unhydrated as well as hydrated forms are preferred. Anticalculus phosphates include potassium and sodium pyrophosphates; sodium tripolyphosphate; diphosphonates, such as ethane-1-hydroxy-1,1-diphosphonate; 1-azacycloheptane-1,1-diphosphonate; and linear alkyl diphosphonates; linear carboxylic acids and sodium and zinc citrate.

Agents that may be used in place of or in combination with the pyrophosphate salt include materials such as synthetic anionic polymers including polyacrylates and copolymers of maleic anhydride or acid and methyl vinyl ether (e.g. Gantrez, as described, for example, in U.S. Patent 4,627, 977, to Gaffar et al. herein incorporated by reference in its entirety, as well as e.g. polyamino propane sulfonic acid (AMPS), zinc citrate trihydrate, polyphosphates (e.g. tripolyphosphate; hexametaphosphate), diphosphonates (e.g. EHDP, AMP), polypeptides (such as polyaspartic and polyglutamic acids), and mixtures thereof.

One of more fluoride ion sources incorporated into the film compositions as anticaries agents. Fluoride ions are included in many oral care compositions for this purpose, and similarly may be incorporated in the invention in the same way. Detailed examples of such fluoride ion sources can be found in US patent 6,121,315 to Nair et al..

Also useful herein are tooth desensitizing agents. Tooth desensitizing agents that may be used include potassium nitrate, citric acid, citric acid salts, strontium chloride, and the like, as well as other desensitizing agents known in the art. The amount of desensitizing agent included within the dental whitening compositions may vary according to the concentration of the potassium nitrates, the desired strength and intended treatment times. Accordingly, if included at all, the other desensitizing agents will preferably be included in an amount in a range from about 0.1 % to about 10% by weight of the dental desensitizing composition, more preferably in a range from about 1 to about 7% by weight of the wet film composition.

Antimicrobial agents can also be present in the film compositions of the present invention as oral agents or topical skin and/or systemic actives. Such agents may include, but are not limited to, 5-chloro-2-(2,4-dichlorophenoxy)- phenol, commonly referred to as triclosan, chlorhexidine, alexidine, hexetidine, sanguinarine, benzaLkonium chloride, salicylamide, domiphen bromide, cetylpyridium chloride (CPC), tetradecyl pyridinium chloride (TPC); N-tetradecyl-4- ethyl pyridinium chloride (TDEPC); octenidine; delmopinol, octapinol, and other piperidino derivatives, niacin preparations; zinc/stannous ion agents; antibiotics such as AUGMENTIN, amoxyicillin, tetracycline, doxycyline, minocycline, and metronidazole; and analogs, derivatives and salts of the above antimicrobial agents and mixtures thereof.

Anti-inflammatory agents can also be present in the film compositions as oral agents or topical skin and/or systemic actives. Such agents may include, but are not limited to, non- steroidal anti-inflammatory agents or NSAIDs, such as propionic acid derivatives; acetic acid derivatives; fenamic acid derivatives; biphenylcarboxylic acid derivatives; and oxicams. All of these NSAIDS are fully described in U.S. Pat. No. 4,985,459 to Sunshine et al., issued Jan. 15, 1991. Examples of useful NSAIDS include acetyl salicylic acid, ibuprofen, naproxen, benoxaprofen, flurbiprofen, fenoprofen, fenbufen, ketoprofen, indoprofen, pirprofen, carprofen, oxaprozin, pranoprofen, microprofen, tioxaprofen, suprofen, alminoprofen, tiaprofenic acid, fluprofen, bucloxic acid and mixtures thereof. Also useful are the steroidal anti-inflammatory drugs such as hydrocortisone and the like, and COX-2 inhibitors such as such as meloxicam, celecoxib, rofecoxib, valdecoxib, etoricoxib or mixtures thereof. Mixtures of any of the above anti-inflammatories may be used.

Anesthetic agent may also be incorporated herein. Examples of suitable anesthetic agents include, but are not limited to, benzocaine, betoxycaine, biphenamine, bupivacaine, butacaine, dibucaine hydrochloride, dyclonine, lidocaine, mepivacaine, procaine, propanidid, propanocaine, proparacaine, propipocaine, propofol, propoxycaine hydrochloride, pseudococaine, tetracaine hydrochloride and mixtures thereof.

Upper respiratory actives can also be used herein. Examples of such actives are sympathomimetic agents administered systemically or topically for decongestant use, including propylhexedrine, phenylephrine, phenylpropanolamine, pseudoephedrine, naphazoline hydrochloride, oxymetazoline hydrochloride, tetrahydrozoline hydrochloride, xylometazoline hydrochloride, and ethylnorepinephrine hydrochloride; anti-histamines are chlorpheniramine, brompheniramine, clemastine, ketotifen, azatadine, loratadine, terfenadine, cetirizine, astemizole, tazifylline, levocabastine, diphenhydramine, temelastine, etolotifen, acrivastine, azelastine, ebastine, mequitazine, mizolastine, levocetirizine, mometasone furoate, carebastine, ramatroban, desloratadine, noberastine, selenotifen, alinastine, efletirizine, tritoqualine, norastemizole, tagorizine, epinastine, acrivastine and mixtures thereof; antitussives such as dextromethorphan, benzonatate, and guifenecin.and mixtures thereof. Other useful upper respiratory actives and be found in US patent 4,619,934.

Gastro-intestinal actives can also be incorporated. Examples of suitable gastro-intestinal actives include anticholinergics, including: atropine, clidinium and dicyclomine; antacids, including aluminum hydroxide, basic bismuth salts such as bismuth subsalicylate, bismuth ranitidine citrate, bismuth subcitrate, bismuth subnitrate, aluminum or bismuth salts of polysulfated saccharides such as aluminum sucrose octasulfate or bismuth sucrose octasulfate, simethicone, calcium carbonate and magaldrate (other examples of antacids can be found in 21CFR 331.11); H (2)-receptor antagonists, including cimetidine, famotidine, nizatidine and ranitidine; laxatives, including: bisacodyl, picosulfate, and casanthrol (other examples of laxatives can be found in the Federal Registry, Vol. 50, No. 10, Jan. 15, 1985, pp. 2152-58); gastroprotectants, including sucralfate and sucralfate humid gel; gastrokinetic and prokinetic agents including cisapride, metoclopramide and eisaprode; proton pump inhibitors including omeprazole, lanzoprazole, and antidiarrheals including: diphenoxylate and loperamide; agents which are bacteriostatic or bactericidal to the ulcer-inducing organism Heliobacter pylori such as amoxicillin, metronidazole, erythromycin, or nitrofurantoin and others agents for treating H. pylori disclosed in U.S. Pat. No. 5,256,684; polyanionic materials useful for the treatment of ulcers and other gastrointestinal disorders including amylopectin, carragemum, sulfated dextrins, inositol hexaphosphate, or other similar agents and mixtures thereof.

Nutrients may improve the condition of the oral cavity and can be included in the oral care substances or compositions of the present invention. Examples of nutrients include minerals, vitamins, oral nutritional supplements, enteral nutritional supplements, and mixtures thereof.

Smoking cessation agents such as nicotine may also be incorporated in the film compositions.

An individual enzyme or combination of several compatible enzymes can also be included in the oral care substance or composition.

Enzymes are biological catalysts of chemical reactions in living devices. Enzymes combine with the substrates on which they act forming an intermediate enzyme substrate complex. This complex is then converted to a reaction product and a liberated enzyme which continues its specific enzymatic function.

Enzymes provide several benefits when used for cleansing of the oral cavity. Proteases break down salivary proteins which are absorbed onto the tooth surface and form the pellicle; the first layer of resulting plaque. Proteases along with lipases destroy bacteria by lysing proteins and lipids which form the structural component of bacterial cell walls and membranes. Dextranases break down the organic skeletal structure produced by bacteria that forms a matrix for bacterial adhesion. Proteases and amylases, not only present plaque formation, but also prevent the development of calculus by breaking-up the carbohydrate protein complex that binds calcium, preventing mineralisation. Enzymes useful in the present invention include any of the commercially available proteases, glucanohydrolases, endoglycosidases, amylases, mutanases, lipases and mucinases or compatible mixtures thereof. Preferred are the proteases, dextranases, endoglycosidases and mutenases, most preferred being papain, endoglycidase, lysozyme or a mixture of dextranase and mutanase.

Other materials that can be used include commonly known mouth and throat products. These products include, but are not limited to anti-fungal, antibiotic and analgesic agents.; Antioxidants are generally recognized as useful in compositions such as those of the present invention. Antioxidants that may be included in the oral care composition or substance include, but are not limited to Vitamin E, ascorbic acid, Uric acid, carotenoids, Vitamin A, flavonoids and polyphenols, herbal antioxidants, melatonin, aminoindoles, lipoic acids and mixtures thereof.

Histamine-(H-2)receptor antagonist compounds (H-2 antagonists) may be used in the oral care composition. As used herein, selective H-2 antagonists are compounds that block H-2 receptors, but do not have meaningful activity in blocking histamine-(H-1) receptors.

Additional useful actives can be found in US patent 6,638,528.

An additional carrier material may also be added to the film composition. These materials can be added as additional components for properties other than those previously mentioned and can include humectants and include glycerin, sorbitol, polyethylene glycol and the like. The film composition may comprise the substance itself, together with one or more substance enhancers, for example catalysts and/or potentiators to modify the release and/or activity of the substance.

The film compositions may additionally comprise additional substances such as flavours, colours, etc. which may for example be deposited onto the surface of the film or impregnated into the bulk of the film. The topical or system active is preferably teeth whitening substance. The teeth whitening substance can take the form of a peroxide-containing gel. Suitable gels may be based on glycerol containing a peroxide such as hydrogen peroxide or an organic peroxide. A suitable gel is that disclosed in US-A-3,657,413, for example that sold under the trade mark PROXIGEL by The Block Drug Company (USA) (since acquired by GlaxoSmithKline plc). Other suitable peroxide-containing gels are for example disclosed in the art references cited above. The film may have the topical or system active deposited upon its surface.

A pH adjusting agent may also be added to optimise the storage stability of the gel and to make the substance safe for the oral tissues. These pH adjusting agents, or buffers, can be any material which is suitable to adjust the pH of the oral care substance. Suitable materials include sodium bicarbonate, sodium phosphate, sodium hydroxide, ammonium hydroxide, sodium stannate, triethanolamine, citric acid, hydrochloric acid, sodium citrate, and combinations thereof. The pH adjusting agents are added in sufficient amounts so as to adjust the pH of the substance or composition to a suitable value, e.g. about 4.5 to about 11, preferably from about 5.5 to about 8.5, and more preferably from about 6 to about 7. The pH adjusting agents are generally present in an amount of from about 0.01 % to about 15% and preferably from about 0.05% to about 5%, by weight of the oral care substance.

### Examples of Film Compositions Disclosed in United States patent applications 11/030,846, and 10/792,362

The film compositions illustrated in following examples illustrate specific embodiments of the film compositions disclosed in United States patent applications 11/030,846, and 10/792,362. Other modifications can be undertaken by the skilled artisan.

All exemplified film compositions can be prepared by conventional formulation and mixing techniques. Component amounts are listed as weight percents and exclude minor materials such as diluents, filler, and so forth. The listed formulations, therefore, comprise the listed components and any minor materials associated with such components.

### Example I

The following is an example of a stand alone film.

| **INGREDIENT** | **AMOUNT** (weight percent) |
|---|---|
| DISTILLED WATER | 10.00 |
| ISO-PROPYL ALCOHOL | 79.00 |
| SILICA (fumed untreated)¹ | 4.00 |
| GLYCERIN USP SPECIAL | 2.00 |
| ZEIN² | 5.00 |

| | |
|---|---|
| ¹ Supplied under the tradename Cabosil® by Cabot, Tuscola, Ill. ² Protein from Corn, (Supplied by Freeman Industries, Tuckahoe, NY). | |

In suitable beaker, zein, silica, alcohol, glycerin and water are mixed until uniform and homogenous.

The contents of the beaker is then cast at desired thickness on a non-stick surface or sheet at room temperature to form the film.

### Example II

The following is an example of a stand alone film.

| **INGREDIENT** | **AMOUNT** (weight percent) |
|---|---|
| ALCOHOL USP/EP | 38.00 |
| SILICA (fumed untreated)¹ | 2.00 |
| CAPOL 150² | 60.00 |

| | |
|---|---|
| ¹ Supplied under the tradename Cabosil® by Cabot, Tuscola, 111. ² Contains Ethanol, Shellac, Hydrogenated Vegetable Oil (Coconut Origin) ( Supplied by Centerchem, Inc., Norwalk, CT). | |

In suitable beaker, Capol 150, silica, and alcohol are mixed until uniform and homogenous.

The contents of the beaker is then cast at desired thickness on a non-stick surface or sheet at room temperature to form the film.

### Example III

The following is an example of a paint -on film forming composition.

| **INGREDIENT** | **AMOUNT** (weight percent) |
|---|---|
| PHARMACEUTICAL GLAZE¹ | 56.00 |
| MAGNESIUM STEARATE² | 3.00 |
| TRIACETIN³ | 1.00 |
| ALCOHOL USP/EP | 40.00 |

| | |
|---|---|
| ¹ Shellac supplied by Mantrose Haeser Co., Attleboro, Ma. ² Magnesium stearate, Hyqual, vegetable source supplied by Mallinckrodt Chemicals, Phillipsburg, NJ. ³ Eastman Triacetin (food grade) supplied by Eastman Chemical Company, Kingsport, TN. | |

In suitable beaker, Pharmaceutical Glaze, magnesium stearate, triacetin, and alcohol are mixed until uniform and homogenous.

The contents of the beaker is then placed in a suitable air-tight container for later application to the skin, teeth, or oral mucosa by the consumer as a paint-on film.

### Example IV

The following is an example of a bi-layer, teeth whitening film.

### Adhesive Layer

| **INGREDIENT** | **AMOUNT (weight percent)** |
|---|---|
| XANTHAN GUM¹ | 0.0174% w/w |
| LOCUST BEAN GUM, CLARIFIED² | 0.0348% w/w |
| CARRAGEENAN³ | 0.1740% w/w |
| PULLULAN⁴ | 4.1000% w/w |
| POVIDONE, USP K-90⁵ | 12.4000% w/w |
| SUCRALOSE⁶ | 0.7000% w/w |
| POTASSIUM PHOSPHATE MONOBASIC NF | 0.0700% w/w |
| PURIFIED WATER, USP/EP | 72.4948% w/w |
| HYDROGEN PEROXIDE 35%⁷ | 5.7100% w/w |
| FLAVOR | 2.5890% w/w |
| POLYSORBATE 80 NF/EP⁸ | 0.3550% w/w |
| EMULSIFIER⁹ | 0.3550% w/w |
| GLYCERIN USP SPECIAL | 1.0000% w/w |

### Backing Layer

| | |
|---|---|
| PHARMACEUTICAL GLAZE, 4-LB CUT NF¹⁰ | 55.0000% w/w |
| SILICA¹¹ (fumed untreated) | 4.0000% w/w |
| ALCOHOL USP/EP | 40.0000% w/w |
| GLYCERYL STEARATE SE¹² | 1.0000% w/w |

| | |
|---|---|
| ¹ Supplied under the name Keltrol T by CP Kelco, Chicago, IL ² Sold under the name Viscogum BCR 20/80 by Degussa Texturant Systems, Atlanta, GA ³ Supplied under the name Viscarin SD339 by FMC Biopolymer, Philadelphia, PA. ⁴ PI-20 grade supplied by Hayashibara. ⁵ Polyvinylpyrrolidone, USP K-90, International Specialties Products(ISP), Wayne, NJ. ⁶ ALB CG 35% hydrogen peroxide solution, Atofina, Philadelphia, Pa. ⁷ Supplied under the tradename Splenda®, by McNeil Pharmaceuticals, NewBrunswick, NJ. ⁸ Tween 80, supplied by Quest, Hoffmann Estates, I11. ⁹ Mixture of mono- and di-oleates supplied under name Atmos 300 by American Ingredients, Kansas City, Mo. ¹⁰ Shellac supplied by Mantrose Haeser Co., Attleboro, Ma. ¹¹ Supplied under the tradename Cabosil® by Cabot, Tuscola, Ill. ¹² Supplied as Mono- and Diglycerides of fats and oils (disposable grade) by Lonza Inc., Fair Lawn, NJ. | |

In a suitable beaker (beaker A), water, sucralose, potassium phosphate monobasic are added with mixing until the mixture is homogenous.

In a separate beaker (beaker B), xanthan gum, locust bean gum, carrageenan, pullulan and Plasdone K-90 are mixed as a dry mix until the mixture is homogenous. The contents of beaker B are mixed into beaker A with rapid mixing or stirring. The combined mixture is mixed until the gums are hydrated. To the combined mixture, the hydrogen peroxide is added slowly with mixing.

In a separate beaker (beaker C), the flavor, polysorbate 80, glycerin and Atmos 300 are mixed until dissolved and uniform. The contents of beaker C are then poured into beaker A and mixed until the mixture is uniform and homogenous. The pH is then adjusted to about 5.5 using 1.0 N sodium hydroxide.

In still another separate beaker (beaker D), the pharmaceutical glaze, Cabosil, alcohol and glyceryl sterate is mixed until uniform and homogenous.

The contents of beaker D is then cast at desired thickness on a non-stick at room temperature to form the inventive film or first layer of the bi-layer, teeth whitening film.

The contents of beaker A is then cast at desired thickness over the above-described first layer at room temperature to form the second layer of the bi-layer, teeth whitening film.

### Example V

The following is an example of a bi-layer, teeth whitening film.

### Adhesive Layer

| **INGREDIENT** | **AMOUNT (weight percent)** |
|---|---|
| XANTHAN GUM¹ | 0.02308% w/w |
| LOCUST BEAN GUM, CLARIFIED² | 0.04616% w/w |
| CARRAGEENAN³ | 0.2308% w/w |
| POVIDONE, USP K-90⁴ | 16.426% w/w |
| SUCRALOSE⁵ | 0.7000% w/w |
| POTASSIUM PHOSPHATE MONOBASIC NF | 0.0700% w/w |
| PURIFIED WATER, USP/EP | 72.4948% w/w |
| HYDROGEN PEROXIDE 35%⁶ | 5.7100% w/w |
| FLAVOR | 2.5890% w/w |
| POLYSORBATE 80 NF/EP⁷ | 0.3550% w/w |
| EMULSIFIER⁸ | 0.3550% w/w |
| GLYCERIN USP SPECIAL | 1.0000% w/w |

### Backing Layer

| | |
|---|---|
| PHARMACEUTICAL GLAZE, 4-LB CUT NF⁹ | 55.0000% w/w |
| SILICA¹⁰ (fumed untreated) | 4.0000% w/w |
| ALCOHOL USP/EP | 40.0000% w/w |
| GLYCERYL STEARATE SE¹¹ | 1.0000% w/w |

| | |
|---|---|
| ¹ Supplied under the name Keltrol T by CP Kelco, Chicago, IL ² Sold under the name Viscogum BCR 20/80 by Degussa Texturant Systems, Atlanta, GA ³ Supplied under the name Viscarin SD339 by FMC Biopolymer, Philadelphia, PA. ⁴ Polyvinylpyrrolidone, USP K-90, International Specialties Products(ISP), Wayne, NJ. ⁵ ALB CG 35% hydrogen peroxide solution, Atofina, Philadelphia, Pa. ⁶ Supplied under the tradename Spenda®, by McNeil Pharmaceuticals, Philadelphia, Pa. ⁷ Tween 80, supplied by Quest, _Hoffmann Estates, 111. ⁸ Mixture of mono- and di-oleates supplied under name Atmos 300 by American Ingredients, Kansas City, Mo. ⁹ Shellac supplied by Mantrose Haeser Co., Attleboro, Ma. ¹⁰ Supplied under the tradename Cabosil® by Cabot, Tuscola, Ill. ¹¹ Supplied as Mono- and Diglycerides of fats and oils (disposable grade) by Lonza Inc., Fair Lawn, NJ. | |

In a suitable beaker (beaker A), water, sucralose, potassium phosphate monobasic are added with mixing until the mixture is homogenous.

In a separate beaker (beaker B), xanthan gum, locust bean gum, carrageenan and Plasdone K-90 are mixed as a dry mix until the mixture is homogenous. The contents of beaker B are mixed into beaker A with rapid mixing or stirring. The combined mixture is mixed until the gums are hydrated. To the combined mixture, the hydrogen peroxide is added slowly with mixing.

In a separate beaker (beaker C), the flavor, polysorbate 80, glycerin and Atmos 300 are mixed until dissolved and uniform. The contents of beaker C are then poured into beaker A and mixed until the mixture is uniform and homogenous. The pH is then adjusted to about 5.5 using 1.0 N sodium hydroxide.

In still another separate beaker (beaker D), the pharmaceutical glaze, Cabosil, alcohol and glyceryl sterate is mixed until uniform and homogenous.

The contents of beaker D is then cast at desired thickness on a non-stick at room temperature to form the inventive film or first layer of the bi-layer, teeth whitening film.

The contents of beaker A is then cast at desired thickness over the above-described first layer at room temperature to form the second layer of the bi-layer, teeth whitening film.

### Example VI

The following is an example of a bi-layer, teeth whitening film.

### Adhesive Layer

| **INGREDIENT** | **AMOUNT (weight percent)** |
|---|---|
| XANTHAN GUM¹ | 0.0674% w/w |
| LOCUST BEAN GUM, CLARIFIED² | 0.0848% w/w |
| PULLULAN³ | 4.1740% w/w |
| POVIDONE, USP K-90⁴ | 12.4000% w/w |
| SUCRALOSE⁵ | 0.7000% w/w |
| POTASSIUM PHOSPHATE MONOBASIC NF | 0.0700% w/w |
| PURIFIED WATER, USP/EP | 72.4948% w/w |
| HYDROGEN PEROXIDE 35%⁶ | 5.7100% w/w |
| FLAVOR | 2.5890% w/w |
| POLYSORBATE 80 NF/EP⁷ | 0.3550% w/w |
| EMULSIFIER⁸ | 0.3550% w/w |
| GLYCERIN USP SPECIAL | 1.0000% w/w |

### Backing Layer

| | |
|---|---|
| PHARMACEUTICAL GLAZE, 4-LB CUT NF⁹ | 55.0000% w/w |
| SILICA¹⁰ (fumed untreated) | 4.0000% w/w |
| ALCOHOL USP/EP | 40.0000% w/w |
| GLYCERYL STEARATE SE¹¹ | 1.0000% w/w |

| | |
|---|---|
| ¹ Supplied under the name Keltrol T by CP Kelco, Chicago, IL ² Sold under the name Viscogum BCR 20/80 by Degussa Texturant Systems, Atlanta, GA ³ PI-20 grade supplied by Hayashibara. ⁴ Polyvinylpyrrolidone, USP K-90, _ International Specialties Products(ISP), Wayne, NJ. ⁵ ALB CG 35% hydrogen peroxide solution, Atofina, Philadelphia, Pa. ⁶ Supplied under the tradename Spenda®, by McNeil Pharmaceuticals, Philadelphia, Pa. ⁷ Tween 80, supplied by Quest, _Hoffmann Estates, Ill. ⁸ mixture of mono- and di-oleates supplied under name Atmos 300 by American Ingredients, Kansas City, Mo. ⁹ Shellac supplied by Mantrose Haeser Co., Attleboro, Ma. ¹⁰ Supplied under the tradename Cabosil® by Cabot, Tuscola, 111. ¹¹ Supplied as Mono- and Diglycerides of fats and oils (disposable grade) by Lonza Inc., Fair Lawn, NJ. | |

In a suitable beaker (beaker A), water, sucralose, potassium phosphate monobasic are added with mixing until the mixture is homogenous.

In a separate beaker (beaker B), xanthan gum, locust bean gum, pullulan and Plasdone K-90 are mixed as a dry mix until the mixture is homogenous. The contents of beaker B are mixed into beaker A with rapid mixing or stirring. The combined mixture is mixed until the gums are hydrated. To the combined mixture, the hydrogen peroxide is added slowly with mixing.

In a separate beaker (beaker C), the flavor, polysorbate 80, glycerin and Atmos 300 are mixed until dissolved and uniform. The contents of beaker C are then poured into beaker A and mixed until the mixture is uniform and homogenous. The pH is then adjusted to about 5.5 using 1.0 N sodium hydroxide.

In still another separate beaker (beaker D), the pharmaceutical glaze, Cabosil, alcohol and glyceryl sterate is mixed until uniform and homogenous.

The contents of beaker D is then cast at desired thickness on a non-stick at room temperature to form the inventive film or first layer of the bi-layer, teeth whitening film.

The contents of beaker A is then cast at desired thickness over the above-described first layer at room temperature to form the second layer of the bi-layer, teeth whitening film.

### Example VII

The following is an example of a bi-layer, teeth whitening film of the present invention.

### Adhesive Layer

| **INGREDIENT** | **AMOUNT (weight percent)** |
|---|---|
| STARCH GUM¹ | 1.9674% w/w |
| GUM ARABIC² | 0.1848% w/w |
| PULLULAN³ | 2.1740% w/w |
| POVIDONE, USP K-90⁴ | 12.4000% w/w |
| SUCRALOSE⁵ | 0.7000% w/w |
| POTASSIUM PHOSPHATE MONOBASIC NF | 0.0700% w/w |
| PURIFIED WATER, USP/EP | 72.4948% w/w |
| HYDROGEN PEROXIDE 35%⁶ | 5.7100% w/w |
| FLAVOR | 2.5890% w/w |
| POLYSORBATE 80 NF/EP⁷ | 0.3550% w/w |
| EMULSFFIER⁸ | 0.3550% w/w |
| GLYCERIN USP SPECIAL | 1.0000% w/w |

### Backing Layer

| | |
|---|---|
| PHARMACEUTICAL GLAZE, 4-LB CUT NF⁹ | 55.0000% w/w |
| SILICA¹⁰ (fumed untreated) | 4.0000% w/w |
| ALCOHOL USP/EP | 40.0000% w/w |
| GLYCERYL STEARATE SE¹¹ | 1.0000% w/w |

| | |
|---|---|
| ¹ Supplied under the trade name of Pure-Cote B760, supplied by Grain processing Corporation, Muscatine, IA. ² Supplied under the name Bright Gum Arabic Spray Dry FCC/NF Powder by TIC Gums, Belcamp, MD ³ PI-20 grade supplied by Hayashibara. ⁴ Polyvinylpyrrolidone, USP K-90, International Specialties Products(ISP), Wayne, NJ. ⁵ ALB CG 35% hydrogen peroxide solution, Atofina, Philadelphia, Pa. ⁶ Supplied under the tradename Spenda®, by McNeil Pharmaceuticals, Philadelphia, Pa. ⁷ Tween 80, supplied by Quest, Hoffmann Estates, III. ⁸ mixture of mono- and di-oleates supplied under name Atmos 300 by American Ingredients, Kansas City, Mo. ⁹ Shellac supplied by Mantrose Haeser Co., Attleboro, Ma. ¹⁰ Supplied under the tradename Cabosil® by Cabot, Tuscola, 111. ¹¹ Supplied as Mono- and Diglycerides of fats and oils (disposable grade) by Lonza Inc., Fair Lawn, NJ. | |

In a suitable beaker (beaker A), water, sucralose, potassium phosphate monobasic are added with mixing until the mixture is homogenous.

In a separate beaker (beaker B), starch gum, gum arabic, pullulan and Plasdone K-90 are mixed as a dry mix until the mixture is homogenous. The contents of beaker B are mixed into beaker A with rapid mixing or stirring. The combined mixture is mixed until the gums are hydrated. To the combined mixture, the hydrogen peroxide is added slowly with mixing.

In a separate beaker (beaker C), the flavor, polysorbate 80, glycerin and Atmos 300 are mixed until dissolved and uniform. The contents of beaker C are then poured into beaker A and mixed until the mixture is uniform and homogenous. The pH is then adjusted to about 5.5 using 1.0 N sodium hydroxide.

In still another separate beaker (beaker D), the pharmaceutical glaze, Cabosil, alcohol and glyceryl sterate is mixed until uniform and homogenous.

The contents of beaker D is then cast at desired thickness on a non-stick at room temperature to form the inventive film or first layer of the bi-layer, teeth whitening film.

The contents of beaker A is then cast at desired thickness over the above-described first layer at room temperature to form the second layer of the bi-layer, teeth whitening film.

### Examples of Film Compositions Disclosed in United States patent application 11/086,517

[0077] The film compositions illustrated in following examples illustrate specific embodiments of the film compositions disclosed in United States patent application 11/086,517. Other modifications can be undertaken by the skilled artisan.

[0078] All exemplified film compositions can be prepared by conventional formulation and mixing techniques. Component amounts are listed as weight percents and exclude minor materials such as diluents, filler, and so forth. The listed formulations, therefore, comprise the listed components and any minor materials associated with such components.

### Example VIII

The following is an example of a stand alone film.

| **INGREDIENT** | **AMOUNT** (weight percent) |
|---|---|
| DISTILLED WATER | 10.00 |
| ISO-PROPYL ALCOHOL | 79.00 |
| EUDRAGIT L100¹ | 4.00 |
| GLYCERIN USP SPECIAL | 1.00 |
| TRIACETIN³ | 1.00 |
| ZEIN² | 5.00 |

| | |
|---|---|
| ¹ Supplied by Röhm Pharma. ² Protein from Corn, (Supplied by Freeman Industries, Tuckahoe, NY). ³ Eastman Triacetin (food grade) supplied by Eastman Chemical Company, Kingsport, TN. | |

In suitable beaker, zein, Eudragit, alcohol, glycerin, Triacetin and water are mixed until uniform.

The contents of the beaker are then cast at desired thickness on a non-stick surface or sheet at room temperature to form the film.

### Example IX

The following is an example of a stand alone film.

| **INGREDIENT** | **AMOUNT** (weight percent) |
|---|---|
| ALCOHOL USP/EP | 37.00 |
| EUDRAGIT L30 D55¹ | 2.00 |
| TRIACETIN² | 1.00 |
| CAPOL 150³ | 60.00 |

| | |
|---|---|
| ¹ Supplied by Röhm Pharma. ² Eastman Triacetin (food grade) supplied by Eastman Chemical Company, Kingsport, TN. ³ Contains Ethanol, Shellac, Hydrogenated Vegetable Oil (Coconut Origin) (Supplied by Centerchem, Inc., Norwalk, CT). | |

In suitable beaker, Capol 150, Eudragit, Triacetin and alcohol are mixed until uniform.

The contents of the beaker are then cast at desired thickness on a non-stick surface or sheet at room temperature to form the film.

### Example X

The following is an example of a paint -on film forming composition.

| **INGREDIENT** | **AMOUNT** (weight percent) |
|---|---|
| PHARMACEUTICAL GLAZE¹ | 56.00 |
| EUDRAGIT L-100² | 3.00 |
| TRIACETIN³ | 1.00 |
| PRAMOXINE⁴ | 1.00 |
| ALCOHOL USP/EP | 39.00 |

| | |
|---|---|
| ¹ Shellac supplied by Mantrose Haeser Co., Attleboro, Ma. ² Supplied by Röhm Pharma ³ Eastman Triacetin (food grade) supplied by Eastman Chemical Company, Kingsport, TN. ⁴ Supplied by . | |

In suitable beaker, Pharmaceutical Glaze, Eudragit, Triacetin, pramoxine, and alcohol are mixed until uniform.

The contents of the beaker are then placed in a suitable air-tight container for later application to the skin, teeth, or oral mucosa by the consumer as a paint-on film.

### Example XI

The following is an example of a bi-layer, teeth whitening film.

### Adhesive Layer

| **INGREDIENT** | **AMOUNT (weight percent)** |
|---|---|
| XANTHAN GUM¹ | 0.0174% w/w |
| LOCUST BEAN GUM, CLARIFIED² | 0.0348% w/w |
| CARRAGEENAN³ | 0.1740% w/w |
| PULLULAN⁴ | 4.1000% w/w |
| POVIDONE, USP K-90⁵ | 12.4000% w/w |
| SUCRALOSE⁶ | 0.7000% w/w |
| POTASSIUM PHOSPHATE MONOBASIC NF | 0.0700% w/w |
| PURIFIED WATER, USP/EP | 72.4948% w/w |
| HYDROGEN PEROXIDE 35%⁷ | 5.7100% w/w |
| FLAVOR | 2.5890% w/w |
| POLYSORBATE 80 NF/EP⁸ | 0.3550% w/w |
| EMULSIFIER⁹ | 0.3550% w/w |
| GLYCERIN USP SPECIAL | 1.0000% w/w |

### Backing Layer

| | |
|---|---|
| PHARMACEUTICAL GLAZE, 4-LB CUT NF¹⁰ | 55.0000% w/w |
| EUDRAGIT L-100¹¹ | 4.0000% w/w |
| ALCOHOL USP/EP | 40.0000% w/w |
| TRIACETIN¹² | 1.0000% w/w |

| | |
|---|---|
| ¹ Supplied under the name Keltrol T by CP Kelco, Chicago, IL ² Sold under the name Viscogum BCR 20/80 by Degussa Texturant Systems, Atlanta, GA ³ Supplied under the name Viscarin SD339 by FMC Biopolymer, Philadelphia, PA. ⁴ PI-20 grade supplied by Hayashibara. ⁵ Polyvinylpyrrolidone, USP K-90, International Specialties Products(ISP), Wayne, NJ. ⁶ ALB CG 35% hydrogen peroxide solution, Atofina, Philadelphia, Pa. ⁷ Supplied under the tradename Splenda®, by McNeil Pharmaceuticals, NewBrunswick, NJ. ⁸ Tween 80, supplied by Quest, Hoffmann Estates, I11. ⁹ Mixture of mono- and di-oleates supplied under name Atmos 300 by American Ingredients, Kansas City, Mo. ¹⁰ Shellac supplied by Mantrose Haeser Co., Attleboro, Ma. ¹¹ Supplied by Röhm Pharma ¹² Eastman Triacetin (food grade) supplied by Eastman Chemical Company, Kingsport, TN. | |

In a suitable beaker (beaker A), water, sucralose, potassium phosphate monobasic are added with mixing until the mixture is uniform.

In a separate beaker (beaker B), xanthan gum, locust bean gum, carrageenan, pullulan and Plasdone K-90 are mixed as a dry mix until the mixture is uniform. The contents of beaker B are mixed into beaker A with rapid mixing or stirring. The combined mixture is mixed until the gums are hydrated. To the combined mixture, the hydrogen peroxide is added slowly with mixing.

In a separate beaker (beaker C), the flavor, polysorbate 80, glycerin and Atmos 300 are mixed until dissolved and uniform. The contents of beaker C are then poured into beaker A and mixed until the mixture is uniform. The pH is then adjusted to about 5.5 using 1.0 N sodium hydroxide.

In still another separate beaker (beaker D), the pharmaceutical glaze, Eudragit, alcohol and triacetin are mixed until uniform.

The contents of beaker D is then cast at desired thickness on a non-stick at room temperature to form the film or first layer of the bi-layer, teeth whitening film.

The contents of beaker A is then cast at desired thickness over the above-described first layer at room temperature to form the second layer of the bi-layer, teeth whitening film.

### Example XII

The following is an example of a bi-layer, teeth whitening film.

### Adhesive Layer

| **INGREDIENT** | **AMOUNT (weight percent)** |
|---|---|
| XANTHAN GUM¹ | 0.02308% w/w |
| LOCUST BEAN GUM, CLARIFIED² | 0.04616% w/w |
| CARRAGEENAN³ | 0.2308% w/w |
| POVIDONE, USP K-90⁴ | 16.426% w/w |
| SUCRALOSE⁵ | 0.7000% w/w |
| POTASSIUM PHOSPHATE MONOBASIC NF | 0.0700% w/w |
| PURIFIED WATER, USP/EP | 72.4948% w/w |
| HYDROGEN PEROXIDE 35%⁶ | 5.7100% w/w |
| FLAVOR | 2.5890% w/w |
| POLYSORBATE 80 NF/EP⁷ | 0.3550% w/w |
| EMULSIFIER⁸ | 0.3550% w/w |
| GLYCERIN USP SPECIAL | 1.0000% w/w |

### Backing Layer

| | |
|---|---|
| PHARMACEUTICAL GLAZE, 4-LB CUT NF⁹ | 55.0000% w/w |
| EUDRAGIT L30 D55¹⁰ | 6.0000% w/w |
| ALCOHOL USP/EP | 38.0000% w/w |
| TRIACETIN¹¹ | 1.0000% w/w |

| | |
|---|---|
| ¹ Supplied under the name Keltrol T by CP Kelco, Chicago, IL ² Sold under the name Viscogum BCR 20/80 by Degussa Texturant Systems, Atlanta, GA ³ Supplied under the name Viscarin SD339 by FMC Biopolymer, Philadelphia, PA. ⁴ Polyvinylpyrrolidone, USP K-90, International Specialties Products(ISP), Wayne, NJ. ⁵ ALB CG 35% hydrogen peroxide solution, Atofina, Philadelphia, Pa. ⁶ Supplied under the tradename Spenda®, by McNeil Pharmaceuticals, Philadelphia, Pa. ⁷ Tween 80, supplied by Quest, _Hoffmann Estates, Ill. ⁸ Mixture of mono- and di-oleates supplied under name Atmos 300 by American Ingredients, Kansas City, Mo. ⁹ Shellac supplied by Mantrose Haeser Co., Attleboro, Ma. ¹⁰ Supplied by Röhm Pharma. ¹¹ Eastman Triacetin (food grade) supplied by Eastman Chemical Company, Kingsport, TN. | |

In a suitable beaker (beaker A), water, sucralose, potassium phosphate monobasic are added with mixing until the mixture is uniform.

In a separate beaker (beaker B), xanthan gum, locust bean gum, carrageenan and Plasdone K-90 are mixed as a dry mix until the mixture is uniform. The contents of beaker B are mixed into beaker A with rapid mixing or stirring. The combined mixture is mixed until the gums are hydrated. To the combined mixture, the hydrogen peroxide is added slowly with mixing.

In a separate beaker (beaker C), the flavor, polysorbate 80, glycerin and Atmos 300 are mixed until dissolved and uniform. The contents of beaker C are then poured into beaker A and mixed until the mixture is uniform. The pH is then adjusted to about 5.5 using 1.0 N sodium hydroxide.

In still another separate beaker (beaker D), the pharmaceutical glaze, Eudragit, alcohol and triacetin are mixed until uniform.

The contents of beaker D is then cast at desired thickness on a non-stick at room temperature to form the film or first layer of the bi-layer, teeth whitening film.

The contents of beaker A is then cast at desired thickness over the above-described first layer at room temperature to form the second layer of the bi-layer, teeth whitening film.

### Example XIII

The following is an example of a bi-layer, teeth whitening film.

### Adhesive Layer

| **INGREDIENT** | **AMOUNT (weight percent)** |
|---|---|
| XANTHAN GUM¹ | 0.0674% w/w |
| LOCUST BEAN GUM, CLARIFIED² | 0.0848% w/w |
| PULLULAN³ | 4.1740% w/w |
| POVIDONE, USP K-90⁴ | 12.4000% w/w |
| SUCRALOSE⁵ | 0.7000% w/w |
| POTASSIUM PHOSPHATE MONOBASIC NF | 0.0700% w/w |
| PURIFIED WATER, USP/EP | 72.4948% w/w |
| HYDROGEN PEROXIDE 35%⁶ | 5.7100% w/w |
| FLAVOR | 2.5890% w/w |
| POLYSORBATE 80 NF/EP⁷ | 0.3550% w/w |
| EMULSIFIER⁸ | 0.3550% w/w |
| GLYCERIN USP SPECIAL | 1.0000% w/w |

### Backing Layer

| | |
|---|---|
| PHARMACEUTICAL GLAZE, 4-LB CUT NF⁹ | 55.0000% w/w |
| ACULYN28¹⁰ | 7.0000% w/w |
| ALCOHOL USP/EP | 37.0000% w/w |
| TRIACETIN¹¹ | 1.0000% w/w |

| | |
|---|---|
| ¹ Supplied under the name Keltrol T by CP Kelco, Chicago, IL ² Sold under the name Viscogum BCR 20/80 by Degussa Texturant Systems, Atlanta, GA ³PI-20 grade supplied by Hayashibara. ⁴ Polyvinylpyrrolidone, USP K-90, _ International Specialties Products(ISP), Wayne, NJ. ⁵ ALB CG 35% hydrogen peroxide solution, Atofina, Philadelphia, Pa. ⁶ Supplied under the tradename Spenda®, by McNeil Pharmaceuticals, Philadelphia, Pa. ⁷Tween 80, supplied by Quest,_Hoffmann Estates, I11. ⁸ mixture of mono- and di-oleates supplied under name Atmos 300 by American Ingredients, Kansas City, Mo. ⁹ Shellac supplied by Mantrose Haeser Co., Attleboro, Ma. ¹⁰ Supplied by Rohm & Haas. ¹¹ Eastman Triacetin (food grade) supplied by Eastman Chemical Company, Kingsport, TN. | |

In a suitable beaker (beaker A), water, sucralose, potassium phosphate monobasic are added with mixing until the mixture is uniform.

In a separate beaker (beaker B), xanthan gum, locust bean gum, pullulan and Plasdone K-90 are mixed as a dry mix until the mixture is uniform. The contents of beaker B are mixed into beaker A with rapid mixing or stirring. The combined mixture is mixed until the gums are hydrated. To the combined mixture, the hydrogen peroxide is added slowly with mixing.

In a separate beaker (beaker C), the flavor, polysorbate 80, glycerin and Atmos 300 are mixed until dissolved and uniform. The contents of beaker C are then poured into beaker A and mixed until the mixture is uniform. The pH is then adjusted to about 5.5 using 1.0 N sodium hydroxide.

In still another separate beaker (beaker D), the pharmaceutical glaze, Aculyn, alcohol and triacetin are mixed until uniform.

The contents of beaker D is then cast at desired thickness on a non-stick at room temperature to form the film or first layer of the bi-layer, teeth whitening film.

The contents of beaker A is then cast at desired thickness over the above-described first layer at room temperature to form the second layer of the bi-layer, teeth whitening film.

### Example XIV

The following is an example of a bi-layer, teeth whitening film.

### Adhesive Layer

| **INGREDIENT** | **AMOUNT (weight percent)** |
|---|---|
| STARCH GUM ¹ | 1.9674% w/w |
| GUM ARABIC² | 0.1848% w/w |
| PULLULAN³ | 2.1740% w/w |
| POVIDONE, USP K-90⁴ | 12.4000% w/w |
| SUCRALOSE⁵ | 0.7000% w/w |
| POTASSIUM PHOSPHATE MONOBASIC NF | 0.0700% w/w |
| PURIFIED WATER, USP/EP | 72.4948% w/w |
| HYDROGEN PEROXIDE 35%⁶ | 5.7100% w/w |
| FLAVOR | 2.5890% w/w |
| POLYSORBATE 80 NF/EP⁷ | 0.3550% w/w |
| EMULSIFIER⁸ | 0.3550% w/w |
| GLYCERIN USP SPECIAL | 1.0000% w/w |

### Backing Layer

| | |
|---|---|
| PHARMACEUTICAL GLAZE, 4-LB CUT NF⁹ | 55.0000% w/w |
| ACULYN 33¹⁰ | 5.0000% w/w |
| ALCOHOL USP/EP | 39.0000% w/w |
| TRIACETIN¹¹ | 1.0000% w/w |

| | |
|---|---|
| ¹ Supplied under the trade name of Pure-Cote B760, supplied by Grain processing Corporation, Muscatine, IA. ² Supplied under the name Bright Gum Arabic Spray Dry FCC/NF Powder by TIC Gums, Belcamp, MD ³ PI-20 grade supplied by Hayashibara. ⁴ Polyvinylpyrrolidone, USP K-90, International Specialties Products(ISP), Wayne, NJ. ⁵ ALB CG 35% hydrogen peroxide solution, Atofina, Philadelphia, Pa. ⁶ Supplied under the tradename Spenda®, by McNeil Pharmaceuticals, Philadelphia, Pa. ⁷Tween 80, supplied by Quest, Hoffmann Estates, III. ⁸ mixture of mono- and di-oleates supplied under name Atmos 300 by American Ingredients, Kansas City, Mo. ⁹ Shellac supplied by Mantrose Haeser Co., Attleboro, Ma. ¹⁰ Supplied by Rohm & Haas. ¹¹ Eastman Triacetin (food grade) supplied by Eastman Chemical Company, Kingsport, TN. | |

In a suitable beaker (beaker A), water, sucralose, potassium phosphate monobasic are added with mixing until the mixture is uniform.

In a separate beaker (beaker B), starch gum, gum arabic, pullulan and Plasdone K-90 are mixed as a dry mix until the mixture is uniform. The contents of beaker B are mixed into beaker A with rapid mixing or stirring. The combined mixture is mixed until the gums are hydrated. To the combined mixture, the hydrogen peroxide is added slowly with mixing.

In a separate beaker (beaker C), the flavor, polysorbate 80, glycerin and Atmos 300 are mixed until dissolved and uniform. The contents of beaker C are then poured into beaker A and mixed until the mixture is uniform. The pH is then adjusted to about 5.5 using 1.0 N sodium hydroxide.

In still another separate beaker (beaker D), the pharmaceutical glaze, Aculyn, alcohol and triacetin are mixed until uniform.

The contents of beaker D is then cast at desired thickness on a non-stick at room temperature to form the film or first layer of the bi-layer, teeth whitening film.

The contents of beaker A is then cast at desired thickness over the above-described first layer at room temperature to form the second layer of the bi-layer, teeth whitening film.

The disclosed films (either single, bi-, or multi-layered) have barrier as well as controlled disintegration properties. The films disclosed in United States patent applications 11/086,517, 11/030,846, and 10/792,362, discussed above, are particularly formed for application to the teeth, oral mucosa, or other affected area of the skin or mouth and allowed to disintegrate over time in the presence of oral fluids or other aqueous media. Examples of oral conditions these substances may address include, but are not limited to, one or more of appearance and structural changes to teeth, whitening, stain bleaching, stain removal, plaque removal, tartar removal, cavity prevention and treatment, inflamed and/or bleeding gums, mucosal wounds, lesions, ulcers, aphthous ulcers, cold sores, tooth abscesses, tooth and/or gum pain, tooth sensitivity (e.g., to temperature changes), and the elimination of mouth malodor resulting from the conditions above and other causes such as microbial proliferation. Additionally, the disclosed films may be useful for treating and/or preventing wounds, lesions, ulcers, cold sores and the like of the lips and skin generally. However, it will be appreciated that a positioning feature formed in accordance with the principles of the present invention may be applied to film products other than simply those disclosed in United States patent applications 11/086,517, 11/030,846, and 10/792,362. Moreover, the principles of the present invention need not be limited by a particular condition to be treated. The positioning features of the present invention are useful for effective use of film products in treating any of a variety of conditions, whether oral or dermal or systemic or otherwise.

Film products to be modified by the present invention may be formed to provide any of a desired variety of functions, such as, without limitation, a therapeutic function. A typical therapeutic function is that of affecting a treatment site, such as by application /delivery of an active to or at such site. Various topical and systemic actives can be incorporated into film products of the present invention, such as disclosed in United States patent applications 11/086,517, 11/030,846, and 10/792,362. The term "topical or system active" as used herein includes, without limitation, curative, prophylactic, and cosmetic active substances or compositions thereof, and includes any actives known in the art. Reference is made to the film products disclosed in United States patent applications 11/086,517, 11/030,846, and 10/792,362 and discussed above for exemplary oral care actives, as well as other inactives, that may be used in the present invention (including suitable topical actives, and levels thereof, for use in and around the oral cavity).

Topical or system actives that may be provided in film products to be used in the present invention may be deposited upon the surface of the film products. For example, a gel may be deposited directly as a layer on a surface of the film product. Alternatively a gel may be absorbed into the film product, or impregnated into the bulk of the film material, or deposited between layers of a multiple layered film.

Methods of depositing substances upon the surfaces of film materials are known, and include, without limitation, printing (e.g., silo screen printing), passing between impregnated rollers, dosing, a pump and nozzle, spraying, dipping, etc. Methods of impregnating substances into the bulk of film materials are also known, and include, without limitation, admixing the substance into the strip material and then forming the strip, or exposure of the strip to the substance under conditions which cause the substance to be impregnated into the strip. Alternatively, one example of the film material may be a foam material, particularly an open-cell foam material, and the substance may be impregnated into the strip material by introducing the substance into the cells of the foam.

In another embodiment, the film forms the first or backing layer of a bilayer where as the second layer is a water soluble polymer film layer such as that described in US patents 6,596,298 to Leung et al. and 6,419,903 to Xu et al. The bilayer film is then applied to the teeth, oral mucosa or other affected area of the skin or mouth and allowed to disintegrate over time in the presence of saliva or other aqueous media.

Additionally the film layers can be manufactured using hot melt extrusion techniques such as that described in US patent 6,375,963 B1 to Repka et al..

As described in United States patent applications 11/086,517, 11/030,846, and 10/792,362, the disclosed film may be marked with one or more visible symbols, e.g., text matter, a trade mark, a company logo, an area of color, or an alignment feature such as a visible line or notch etc. to assist the user in applying the device to the teeth in a proper alignment. Such an alignment feature may, for example, comprise a symbol to show the user which way up the product should be whilst applying the product to the teeth (i.e. which product configuration is "up"), or which of a pair of the products is intended for the upper teeth and which for the lower teeth. Such an alignment feature may also, for example, comprise a symbol to show the user which side of a pair of opposite sides comprises the active or a particular active. This way, the product may be made more visually attractive and/or easier to use and/or more effective.

Such symbol(s) may be applied by conventional printing or embossing processes (e.g., silk screen printing, inkjet printing, etc.) to the surface of the plastically deformable material opposite to the surface on which is attached the layer of an absorbent material. If such a visible symbol is applied to this surface, a cover layer can, optionally, be applied over the symbol, for example to protect it. This cover layer may be transparent or translucent to allow visible symbols to be seen through this layer. Such a cover layer can, optionally, be applied to the film by pressing, e.g., rolling, the material of the cover layer in contact with the film.

The present invention elucidates and elaborates upon the provision of an alignment or positioning feature as disclosed in United States patent applications 11/086,517, 11/030,846, and 10/792,362. The positioning feature preferably is formed and/or located to aid the user in differentiating or distinguishing features of the film product so that the film product may be used efficaciously. For instance, use of the positioning feature to identify a feature of the film product may aid in positioning the film product to achieve its desired effect or function, such as by orienting the film product either independently of and prior to application to the treatment site, or during application to and with respect to the treatment site. Other functions may be performed or achieved by the use of a positioning feature in accordance with the principles of the present invention, the scope of the invention not being limited by the specific function of the film product or by the described exemplary orienting function of the positioning feature.

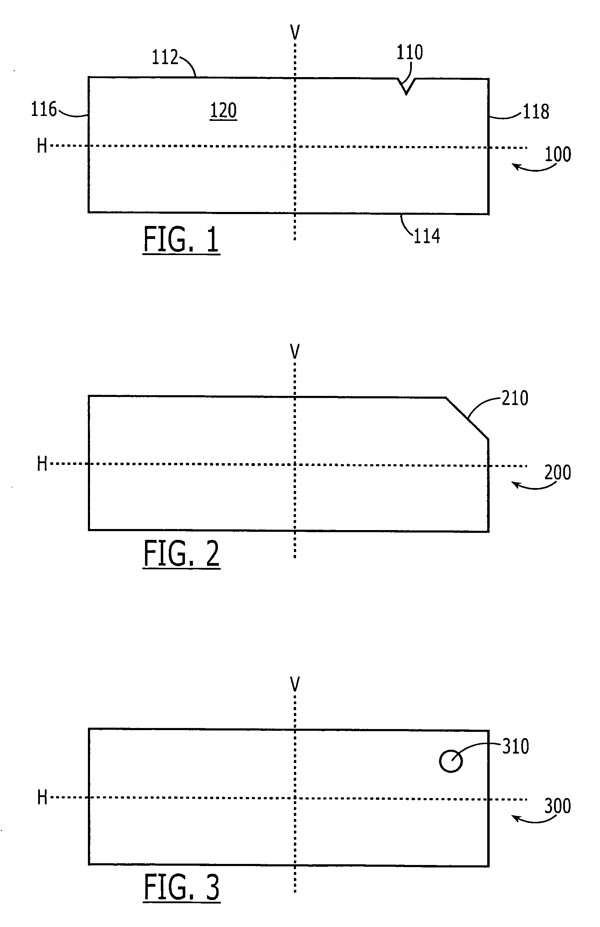

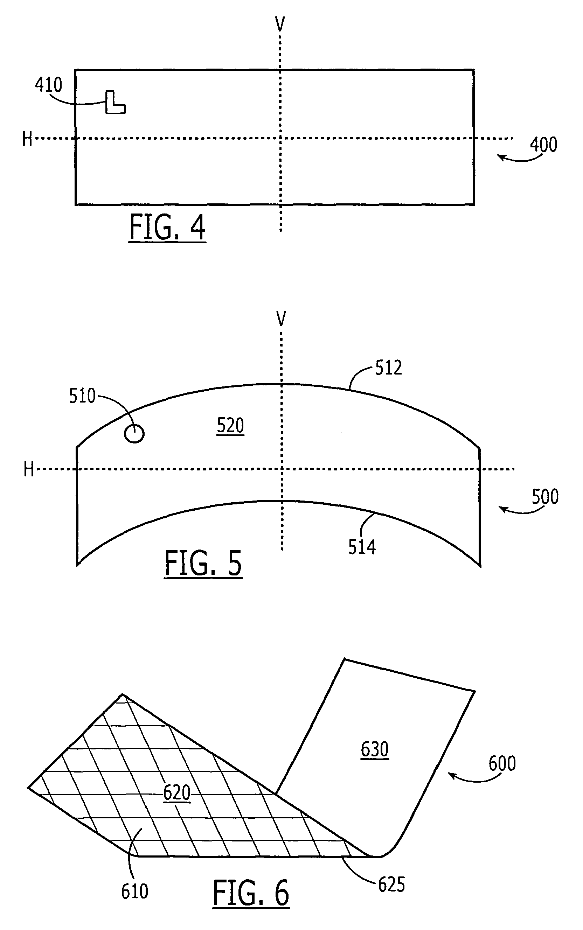

Turning to the drawings, exemplary film products **100, 200, 300, 400, 500**, and **600** are illustrated in FIGURES 1-6 with exemplary respective positioning features **110, 210, 310, 410, 510**, and **610**. In the following description, elements or components similar to those in the embodiment of Figs. 1 to 6 (though not necessarily having identical features) are designated with the same reference numbers increased by **100** for each increase in figure number and redundant description is omitted. It will be appreciated that the film product may be in any desired shape or form, and may be made of any desired material or composition. Thus, although the exemplary film products **100, 200, 300,** and **400** illustrated in FIGURES 1-4 are rectangular, it will be appreciated that the principles of the present invention may be applied to a film product of any other desired shape, such as round, square, triangular, trapezoidal, irregular, etc. For instance, a film product may be formed with curvilinear sides, such as film product **500** of FIGURE 5.

It will further be appreciated that the positioning feature to be provided on a film product in accordance with the principles of the present invention may be in any desired shape or form that achieves the desired purpose of distinguishing or differentiating features or aspects of the film product for proper use and/or application and/or functioning of the film product. For instance, the positioning feature preferably may be provided to identify a particular orientation of the film product itself, such as by identifying which surface is facing the user and which surface is facing the treatment site. The positioning feature may alternatively or additionally be provided simply for purposes of alignment at and/or with respect to the treatment site. The manner in which the positioning feature is provided, such as the form or location, need not be limited to the exemplary embodiments of FIGURES 1-6. Instead, it will be appreciated that the basic function of the positioning feature as distinguishing or differentiating features or aspects of the film product may be achieved without restricting the precise form of the positioning feature.

Turning to the exemplary embodiments of FIGURES 1-6, a positioning feature formed in accordance with the principles of the present invention may be formed in any of a variety of manners, including, without limitation, a mechanical / structural identifier, a visual indicator on a surface, or a textural feature on a surface. The film product may be provided with a positioning feature by being shaped or marked with a shape, cut-out, figure, color, hologram, mark, word, texture, or other indicia, which can uniquely identify sufficient information about the film product for the desired manipulation and/or orienting and/or general use of the film product. The type of positioning feature, as well as its location, may dictate the feature-distinguishing and indicating functions the positioning feature serves.

One simple embodiment of a positioning feature in accordance with the principles of the present invention is a mechanical or structural identifier (hereinafter, simply "mechanical identifier" for the sake of convenience and without intent to limit such term) such as positioning features **110, 210, 310, 410, 510** of FIGURES 1-5. Positioning features **110, 210, 310, 410, 510** are formed by mechanically altering the structure of the film product **100, 200, 300, 400, 500**. For instance, an irregularity may be formed along an edge of the film product, as in FIGURES 1 and 2, or a cut-out may be formed through the film product spaced from the edges of the film product, as in FIGURES 3-5. More particularly, an irregularity along an edge of the film product may be formed in any desired shape, such as a notch **110** (FIGURE 1) or a cut-off corner **210** (FIGURE 2). Similarly, a cut-out may be formed in any desired shape, such as a round hole **310, 510** (FIGURES 3 and 5) or hole of another shape, such as an asymmetrical shape like L-shaped hole **410** (FIGURE 4).

Positioning features in the form of a mechanical identifier by their very nature can indicate orientation of the film product with respect to a treatment site. However, a simple mechanical identifier cannot necessarily definitively indicate the orientation of the surfaces of the film product itself. This is because the presence alone of such an indicator as a mechanical identifier may not provide sufficient data to a user to be able to differentiate at least certain features of the film product. For example, if a film product is symmetrical about an axis of symmetry, then a mechanical identifier positioned along such axis of symmetry (especially a mechanical identifier that itself is symmetrical about such axis of symmetry as well) cannot necessarily serve to differentiate the opposing surfaces of the film product from each other. As another example, a round hole provided as a positioning feature in a round film product would not necessarily provide sufficient information to differentiate the opposing surfaces of the film product.

One manner of allowing a positioning feature in the form of a mechanical identifier to differentiate, more definitively, the surfaces of a film product is to locate the mechanical identifier at a readily identifiable position on the film product itself. Generally, the ability of a mechanical identifier to distinguish a surface of a film product is facilitated by basing selection of the location of the mechanical identifier on the shape of the film product. One useful principle is to place the mechanical identifier offset from any and all axes of symmetry. As discussed above, if the mechanical identifier is along an axis of symmetry of a film product it is difficult, if not impossible, to differentiate the opposite surfaces of the film product. Another useful principle is that the shape of the mechanical identifier can serve as a further indicator of the orientation of the film product on which the mechanical identifier is provided. Such principles may be better appreciated with reference to the exemplary embodiments of FIGURES 1-4, as will now be described.

Exemplary film products **100, 200, 300**, and **400** have shapes that are symmetrical about a horizontal central axis and also about a vertical central axis. Accordingly, it is desirable to locate a positioning feature **110, 210, 310, 410** in the form of a mechanical identifier offset from both the horizontal and vertical central axes so that the mechanical identifier may serve to differentiate the symmetrical sides of the film product. Reference is made to film product **100** of FIGURE 1 to illustrate this principle. Film product **100** has two pairs of opposite edges: opposite edges **112** and **114** (respectively top and bottom edges according to the orientation of film product **100** in FIGURE 1), and opposite edges **116** and **118** (respectively left and right edges according to the orientation of film product **100** in FIGURE 1). Positioning feature **110** is formed along top edge **112** at a location offset from central vertical axis **V**. In other words, positioning feature **110** is not located at a midpoint along the length of top edge **112**. Moreover, because top edge **112** is, by its very nature as an edge, offset from central horizontal axis **H**, positioning feature **110**, when provided along top edge **112**, necessarily is also offset from central horizontal axis H as well. Accordingly, a user may be assured that when looking at film product **100** if positioning feature **110** is on the right side of top edge **112** of film product **100**, then the surface **120** seen in FIGURE 1 is the surface facing the user at that point. Rotation of film product **100** within the plane of the page does not alter such clear position indicating capability. For instance, if positioning feature **110** is located along what appears to be a bottom edge, on the left side thereof, it is also clear that surface **120** is facing the user. In contrast, if positioning feature **110** appears on the left side of top edge **112**, then it is clear to the user that the surface opposite surface **120** is facing the user.

In contrast, exemplary film product **500** of FIGURE 5 illustrates a film product that is symmetrical about only one axis of symmetry (vertical central axis **V**). The asymmetrical sides of film product **500** are inherently distinguishable from one another. Accordingly, positioning feature **510** need only be positioned offset from the axis of symmetry, axis **V**. So long as positioning feature **510** appears on the left side of film product **500** when convex edge **512** is a "top" edge and concave edge **514** is a "bottom" edge, it is clear that the surface **520** of film product **500** seen in FIGURE 5 is the surface facing the user at that point as well.

Of course, complete symmetry, such as in a circular film product, complicates placement, as described above with respect to provision of a round hole as the positioning feature for a round film product. An asymmetrical positioning feature, such as the L-shaped positioning feature **410** of film product **400** in FIGURE 4, may be used advantageously to permit orientation of the asymmetrical positioning feature to distinguish symmetrical sides from one another and thus to permit differentiation of the surfaces of a symmetrical film product.

As noted above, instead of providing a positioning feature in the form of a mechanical identifier, a positioning feature may be formed in accordance with the principles of the present invention as a surface feature which alters the surface of the film product. If the positioning feature is formed on a surface of the film product, then such positioning feature is helpful in differentiating opposing surfaces of the film product. In general, in contrast with a structural feature, a surface feature preferably does not extend through the material of the film product. Such a surface feature may be in any desired form that permits either visual or tactile differentiation of the surfaces of the film product. For instance, a visual indicator such as visible symbols, printed indicia, stippling, shading, or coloring may be used for ready visual differentiation of the surface bearing such visual indicator. Such visual indicator may be applied by any desired technique, including, without limitation, blotching, coloring, cutting, embossing, engraving, marking, printing (by ink jet, video jet, or flexographic printing, or any other desired technique known in the art), shaping, stamping. Additionally, or alternatively, surface texturing that alters the tactile features of the surface, such as by formation of non-smooth, unsmooth, or rough areas or regions ("textured" hereafter for the sake of convenience and without intent to limit), may be used to differentiate surfaces of the film product via tactile sensation.

Positioning features in the form of surface features are generally useful for film products that carry a particular active on only one surface thereof and only deliver the active at or via that surface. Such positioning feature may be particularly helpful in instances in which the surfaces are not readily distinguishable from each other. Examples of film products having surfaces that are not readily distinguishable include film products utilizing a dry adhesive such that a releasable backing strip is not needed to protect a sticky adhesive surface and thus cannot provide an indication of which surface bears the adhesive and is to be applied to the treatment site.

An exemplary embodiment utilizing a surface feature as a positioning feature is illustrated in FIGURE 6. Film product **600** of FIGURE 6 is illustrated folded over itself to show opposite surfaces **620** and **630**. As may be appreciated, positioning feature **610** is a surface feature provided on only one of the surfaces of film product **600**. As such, surface **620**, bearing positioning feature **610**, is readily distinguishable from surface **630**.

Thus, it will be appreciated that the present invention facilitates use and/or application of a film product upon the user's location or identification of a positioning feature provided on the film product in accordance with the principles of the present invention. Once the positioning feature has been located or identified, the user can now differentiate or distinguish at least one feature of the film product. Such information may be employed by the user to use and/or manipulate and/or orient and/or apply the film product as desired. Film products incorporating positioning features in accordance with the principles of the present invention may be sold with instructions for the end users regarding locating the positioning feature, and identifying a desired feature of the film product based on information obtainable upon locating the positioning feature. The instructions may further provided guidance as to use of the film product in conjunction with location of the positioning feature.

As discussed earlier, it will be appreciated that the film products **100, 200, 300, 400, 500** may be any type of film product preferably configured to provide a therapeutic affect at a desired treatment site. One example of a film product that may embody the principles of the present invention is a medicated strip for delivering a systemic or topical active to a treatment site. A more specific example of such a medicated strip is one applied to teeth to whiten the teeth. The positioning features of the present invention are particularly helpful in assisting the user in orienting the medicated strip for proper application to his/her teeth. One exemplary tooth whitening strip utilizes a dry, moistenable adhesive and therefore can be applied directly to the user's teeth upon removal from a protective packaging or pouch without the need to remove a releasable backing strip or otherwise to alter the structure or material of the tooth whitening strip. Provision of a positioning feature on the tooth whitening strip facilitates orientation of the strip, which is essential if only one of the surfaces can deliver the whitening agent. It will, however, be appreciated that the principles of the present invention may be applied to film or strip products for use other than in oral cavities.

It will be appreciated that features described with respect to one embodiment typically may be applied to another embodiment, whether or not explicitly indicated. The various features hereinafter described may be used singly or in any combination thereof. Therefore, the present invention is not limited to only the embodiments specifically described herein. Moreover, the film compositions described in the examples disclosed in United States patent applications 11/086,517, 11/030,846, and 10/792,362 as well as herein illustrate specific embodiments of the film compositions of the present invention, but are not intended to be limiting thereof. Other modifications can be undertaken by the skilled artisan without departing from the scope of the present invention

While the foregoing description and drawings represent exemplary embodiments of the present invention, it will be understood that various additions, modifications, and substitutions may be made therein without departing from the scope of the present invention. In particular, it will be clear to those skilled in the art that the present invention may be embodied in other specific forms, structures, arrangements, proportions, and with other elements, materials, and components, without departing from the spirit or essential characteristics thereof. One skilled in the art will appreciate that the principles of the present invention may be used with many modifications of structure, arrangement, proportions, materials, and components and otherwise, used in the practice of the invention, which are particularly adapted to specific environments and operative requirements without departing from the principles of the present invention. The presently disclosed embodiments are therefore to be considered in all respects as illustrative and not restrictive, the scope of the invention being indicated by the appended claims, and not limited to the foregoing description.

## Claims

1. An article for delivering at least one active to a treatment site on a body, said article comprising:
a film product configured to deliver at least one active to the treatment site; and
a positioning feature provided on said film product to distinguish said film product orientation for positioning said film product at said treatment site in a selected orientation.

2. An article for providing a therapeutic affect at a treatment site, said article comprising:
a film product configured for positioning on a body area to provide the therapeutic affect to said body area; and
a positioning feature provided on said film product to distinguish said film product orientation for positioning on said body area in a selected orientation.

3. An article as in claim 1 or 2, wherein:
said film product has first and second opposing surfaces that are not readily distinguishable from each other; and
said positioning feature is provided to facilitate differentiating said first opposing surface from said second opposing surface of said film product.

4. An article as in claim 2 or 3, wherein said positioning feature comprises a surface feature on at least one of said first and second opposing surfaces.

5. An article as in claim 4, wherein said surface feature permits visual or tactile differentiation of said first and second opposing surfaces.

6. An article as in claim 2 or 3, wherein said positioning feature comprises a mechanical identifier that structurally alters said film product to distinguish at least one feature of said film product.

7. An article as in claim 1 or 2, wherein said positioning feature is provided offset from any lines of symmetry of said film product.

8. An article as in claim 1 or 2, wherein said positioning feature is asymmetrical in shape.

9. An article as in claim 2, wherein:
said film product has first and second opposing surfaces;
said film product comprises a film composition carrying at least one active on only one of said first and second opposing surfaces;
said first and second opposing surfaces are not readily distinguishable from each other; and
said positioning feature serves to distinguish said first and second opposing surfaces from each other.

10. A method of applying a film product to a treatment site, said method comprising:
locating a positioning feature on said film product; and
utilizing said positioning feature to orient said film product for application to said treatment site.

11. A method as in claim 10, further comprising orienting said film product to contact only one of two opposing surfaces of said film product to the treatment site.

12. A method as in claim 10, further comprising orienting said film product with respect to the treatment site.

13. A method as in claim 10, further comprising applying said film product to said treatment site without altering said film product.

14. A method as in claim 13, wherein altering said film product includes removing or adding a material from or to said film product.
